# EUROPEAN PATENT APPLICATION

(11) **EP 3 437 554 A1**
(43) Date of publication of application: **06.02.2019**
(21) Application number: 17184480.6
(22) Date of filing: 02.08.2017
(51) Int. Cl.: A61B 5/05

(54) **DEVICE AND METHOD FOR DETECTING ATRIAL FIBRILLATION OF A SUBJECT**

(71) Applicant: Koninklijke Philips N.V., 5656 AE Eindhoven (NL)
(72) Inventor: MUEHLSTEFF, Jens, 5656 AE Eindhoven (NL); BONOMI, Alberto Giovanni, 5656 AE Eindhoven (NL)
(74) Representative: de Haan, Poul Erik

(57) **Abstract**

The present invention relates to a device, system and method for detecting atrial fibrillation of a subject. To improve detection performance the device comprises a radar input (31) configured to obtain a radar signal acquired by a radar sensor arranged at the subject's thorax, said radar signal representing radiation reflected from the subject's thorax in response to radiation emitted to the subject's thorax, a signal input (32) configured to obtain at least one a reference signal of the subject, said reference signal representing an electrocardiography, ECG, signal, a photoplethysmography, PPG, signal or an accelerometer, ACC, signal and being acquired synchronously with the radar signal, and an analysis unit (33) configured to identify p-wave time intervals and/or ventricle contraction intervals in the reference signal and to evaluate one or more p-wave sections of the radar signal corresponding to identified p-wave time intervals and/or one or more ventricle contraction sections of the radar signal corresponding to identified ventricle contraction intervals to detect atrial fibrillation of the subject.

## Description

### FIELD OF THE INVENTION

The present invention relates to a device, system and method for detecting atrial fibrillation of a subject, e.g. of a person such as a patient.

### BACKGROUND OF THE INVENTION

Atrial fibrillation (AF) is a cardiac condition in which the heart's electrical signal during polarization / depolarization does not travel through the heart in a normal way. Instead, the electrical excitations become very rapid, disorganized and finally cause the heart's atria to fibrillate, or contract very fast and irregular. This condition results in an insufficiently filled ventricle typically accompanied with lower stroke volume during the heart phase. It is a significant issue in all societies due to a high prevalence and incidence. There are about 5 million US inhabitants with AF.

If AF is undetected, untreated AF can have severe consequences such a stroke or heart failure. The risk to suffer from a stroke for a person with AF is six times higher than in healthy subjects. Due to the compromised excitation of the heart muscle, the mechanical movements of the heart wall are different compared to the normal heart.

Currently most monitoring solutions for detecting AF are only based on ECG measurements. This technology focusses on two particular features being detected:
i) irregularities in inter-beat intervals; and
ii) detection of the presence / disappearance of the p-wave in the ECG signal trace.

The reliable detection of the p-wave is a challenge in low lead ECG setups or for unusual non-standardized ECG-lead applications.

The performance achieved via this ECG approach show that a reduced number of ECG leads, when only the inter-beat (RR-intervals) can be obtained, is characterized by a high false positive detection rate, which make AF detection more burdensome for the medical community as more resources are needed to run follow-up procedures for each falsely detected AF patients.

Due to the economic burden and the inherent severe consequences of undetected AF, methods in reliable detection of AF have been investigated. Examples of conventional solutions are single-lead ECG patch devices, handheld devices (also based on ECG) or PPG-based solutions, e.g. the implementation of a detector using the flashlight and the camera of a smartphone.

To improve diagnostic yield in AF detection it would be advantageous to objectify heart wall motion changes.

### SUMMARY OF THE INVENTION

It is an object of the present invention to provide a device (in particular a wearable or handheld device), system and method for detecting atrial fibrillation of a subject with improved detection performance compared to conventional solutions.

In a first aspect of the present invention a device for detecting atrial fibrillation of a subject is presented comprising:
- a radar input configured to obtain a radar signal acquired by a radar sensor arranged at the subject's thorax, said radar signal representing radiation reflected from the subject's thorax in response to radiation emitted to the subject's thorax,
- a signal input configured to obtain at least one reference signal of the subject, said reference signal representing an electrocardiography, ECG, signal, a photoplethysmography, PPG, signal or an accelerometer, ACC, signal and being acquired synchronously with the radar signal,
- an analysis unit configured to identify p-wave time intervals and/or ventricle contraction intervals in the reference signal and to evaluate one or more p-wave sections of the radar signal corresponding to identified p-wave time intervals and/or one or more ventricle contraction sections of the radar signal corresponding to identified ventricle contraction intervals to detect atrial fibrillation of the subject.

In a further aspect of the present invention a system for detecting atrial fibrillation of a subject is presented comprising:
- a radar sensor configured to acquire a radar signal when arranged at the subject's thorax, said radar signal representing radiation reflected from the subject's thorax in response to radiation emitted to the subject's thorax,
- a reference signal sensor configured to acquire a reference signal of the subject, said reference signal representing an electrocardiography, ECG, signal, a photoplethysmography, PPG, signal or an accelerometer, ACC, signal and being acquired synchronously with the radar signal,
- a device as disclosed herein for detecting atrial fibrillation of a subject based on the acquired radar signal and the acquired reference signal.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the recognition that conventional single-lead ECG based detection systems suffer from performance issues due to large false positive rates, which is due to difficult assessment of p-waves in single or two lead ECG based monitoring devices. Conventional technologies used to monitor intra-thoracic motion and multi-lead ECG cannot be adapted for use in a non-clinical environment due to size, costs and principle obstacles in application (like the need of contact gel in ultrasound (US)). These technologies are also difficult to design for handling by non-professionals.

The present invention enables an earlier detection of silent AF by the combination of at least two complementary sensing modalities including radar (providing a radar signal) and one or more of ECG, PPG and ACC (providing a reference signal). From the signals acquired via these sensing modalities p-wave time intervals and/or ventricle contraction intervals are identified in the reference signal. One or more p-wave sections of the radar signal corresponding to identified p-wave time intervals are then evaluated to detect atrial fibrillation of the subject. Alternatively or in addition one or more ventricle contraction sections of the radar signal corresponding to identified and assessed ventricle contraction intervals may be evaluated to detect atrial fibrillation of the subject.

Due to a better sensitivity and specificity detecting AF compared to conventional techniques based on single-lead ECG measurements alone, a lower false positives rate can thus be achieved. Further, the proposed solution enables improved performance to detect other heart arrhythmias with improved detection performance compared to known solutions. Patients are supported during cardiac rehabilitation via objective measures of the patient's heart performance, and the patient and caregiver are warned early and reliably by alarms in case of critical states. The invention is easy to implement and use, e.g. in small wearable, handheld or mobile (e.g. battery-operated) devices, which can also be applied by untrained person.

The proposed solution thus overcomes the following disadvantages of conventional approaches:
- Lower performance of AF detection algorithms based on single-lead ECG; currently RR-intervals provide indications of AF presence but with relatively high false positives.
- Multi-lead ECG is typically not feasible for long-term heart monitoring in home settings due to compromised comfort, however long-term monitoring is required to detect silent and transient AF, since spot checks have very low diagnostic yield.
- There is currently no objectified assessment of heart wall motion as indicator of pathological heart wall motion due to AF with conventional technologies such as ultrasound, CT or MR.
- Ultrasound (US) is not feasible due to the fact that US is attenuated by bones.

The present invention thus allows an easy-to-use monitoring device, system and method that can be produced at relatively low cost for detecting AF with higher detection performance compared to conventional devices, systems and methods suitable for long-term home monitoring.

Generally, the p-wave approach is only relevant for ECG analysis, since a missing p-wave shows irregular electrical activation of the heart contraction. From PPG and ACC signals changes of the contraction process of the heart, i.e. ventricle contraction sections, can be identified. PPG signals allow segmentation of systolic and diastolic phases and ACC signals allow segmentation of contractions, valve openings and valve closures.

The various modalities each have advantages and disadvantages. ECG requires to place electrodes on the body and is subject to motion artifacts as well as the SNR depends on the contact quality, however, this signal is widely accepted. PPG is a well-known technology, requires only a single spot and is available at very low cost; still it is very prone to motion artifacts and contact pressure dependent; the signal represents the blood volume change of the lower capillary bed and not the larger arteries. ACC provide a mechanical measure (much closer to the radar signal), and low cost calibrated and sensitive devices are available at low power, but it is highly sensitive to motion and a non-standard signal (still close to heart sounds).

According to a preferred embodiment said reference signal is an ECG signal and said analysis unit is configured to evaluate one or more p-wave sections of the radar signal by comparing the p-wave sections to a p-wave section template or baseline and by using changes in signal morphology and/or of typical features of the radar signal to detect atrial fibrillation of the subject. The p-wave section template or baseline is predetermined for a healthy subject and e.g. stored in the device or obtained from a database. Different p-wave section templates or baselines may be available for different subjects, e.g. subjects of different gender, age, size, weight, past medical history, etc., i.e. the most appropriate p-wave section template or baseline may then be selected for the analysis based on subject data of the current subject under examination.

The device may further comprise a posture input configured to obtain a posture signal indicating posture of the subject during the acquisition of the radar signal, wherein said analysis unit is configured to select, based on the obtained posture signal, a p-wave section template or baseline from a number of p-wave section templates or baselines for different postures. Radar signatures, i.e. changes in signal morphology and/or typical features of the radar signal, generally depend on the posture of the subject since the heart can move internally so that distances from the radar sensor to the heart changes causing changes of the radar signatures as well. To improve reliability of the detection the posture of the subject is preferably included in the signal processing chain as proposed in this embodiment.

For this purpose the device may further comprise a posture determination unit configured to determine said posture signal from an ACC signal obtained as reference signal. Posture information may e.g. be derived from the accelerometer by detecting the gravity vector projected on the axis of the ACC sensors. If the orientation of the ACC in respect to the subject is known, the posture can be inferred.

In another embodiment said reference signal is an ECG signal and said analysis unit is configured to evaluate one or more p-wave sections of the radar signal by comparing a recent p-wave section to one or more earlier p-wave sections and by using changes in signal morphology and/or of typical features of the radar signal to detect atrial fibrillation of the subject.

According to other embodiment said reference signal is a PPG signal or an ACC signal. In this case the analysis unit may be configured to evaluate one or more ventricle contraction sections of the radar signal by comparing the ventricle contraction sections to a ventricle contraction section template or baseline and by using changes in signal morphology and/or of typical features of the radar signal to detect atrial fibrillation of the subject, or the analysis unit may be configured to evaluate one or more ventricle contraction sections of the radar signal by comparing a recent ventricle contraction section to one or more earlier ventricle contraction sections and by using changes in signal morphology and/or of typical features of the radar signal to detect atrial fibrillation of the subject.

Hereby, said analysis unit may be configured to identify systolic phases and/or diastolic phases as ventricle contraction intervals in case the reference signal is a PPG signal and to identify contraction phases and/or valve opening phases and/or valve closure phases as ventricle contraction intervals in case the reference signal is an ACC signal.

It may also be advantageous to evaluate two or more different kinds of reference signals in order to improve the reliability of the AF detection. Accordingly, in an embodiment said analysis unit is configured to identify p-wave time intervals in an ECG signal representing a first reference signal and to identify ventricle contraction intervals in a PPG signal or an ACC signal representing a second reference signal and to evaluate one or more p-wave sections of the radar signal corresponding to identified p-wave time intervals and one or more ventricle contraction sections of the radar signal corresponding to identified ventricle contraction intervals to detect atrial fibrillation of the subject.

The sensors are preferably configured as wearable or handheld sensors, optionally combined into a common sensor unit. To find the optimum location where to mount or hold the sensors it is proposed in another embodiment said radar input is configured to obtain radar signals acquired at different locations of the subject's thorax and/or said signal input is configured to obtain reference signals acquired at different locations of the subject's thorax, wherein said device further comprises a recommendation unit configured to evaluate the obtained radar signals and/or the obtained reference signals to identify and recommend the optimum location of the subject's thorax for arrangement of the radar sensor for acquiring radar signals and one or more reference sensors for acquiring one or more reference signals for use in the detection of atrial fibrillation. This will improve the quality and reliability of the AF detection.

As mentioned the radar sensor and the one or more reference sensor(s) may be combined into a single sensor unit. The above described device for receiving and evaluating the signals acquired by these sensors may be arranged separately/remotely, i.e. may e.g. be a computer, processor, or other processing unit included in another device, e.g. in a smartphone. In this case the signals acquired by these sensors are transmitted, wirelessly or in through a wire, to the device. In another embodiment, all elements of the disclosed system, i.e. the radar sensor and the one or more reference sensor(s) and the device, may be implemented as wearable or handheld device that can be mounted to the subject's body or that can be held at the subject's body for detecting atrial fibrillation of the subject. It is thus possible for a subject to walk around with the device and use it e.g. at home for self diagnosis.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a first embodiment of a system for detecting atrial fibrillation of a subject according to the present invention,
Fig. 2 shows a schematic diagram of a first embodiment of a device for detecting atrial fibrillation of a subject according to the present invention,
Fig. 3 shows a schematic diagram of a second embodiment of a system for detecting atrial fibrillation of a subject according to the present invention,
Fig. 4 shows a signal diagram of a radar signal and an ECG signal as used in the system shown in Fig. 3,
Fig. 5 shows a schematic diagram of a second embodiment of a device for detecting atrial fibrillation of a subject according to the present invention,
Fig. 6 shows a flowchart of a method for acquiring radar signal templates,
Fig. 7 shows a flowchart of a first embodiment of a method according to the present invention,
Fig 8 shows a flowchart of a second embodiment of a method according to the present invention,
Fig. 9 shows a signal diagram of an ECG signal and an ACC signal as used in another embodiment of the device and system according to the present invention,
Fig. 10 shows a signal diagram of another ECG signal and another ACC signal as used in an embodiment of the device and system according to the present invention,
Fig. 11 shows a signal diagram of an ECG signal and a PPG signal as used in another embodiment of the device and system according to the present invention, and
Fig. 12 illustrates another embodiment of a device according to the present invention enabling optimized placement of the sensors.

### DETAILED DESCRIPTION OF THE EMBODIMENTS

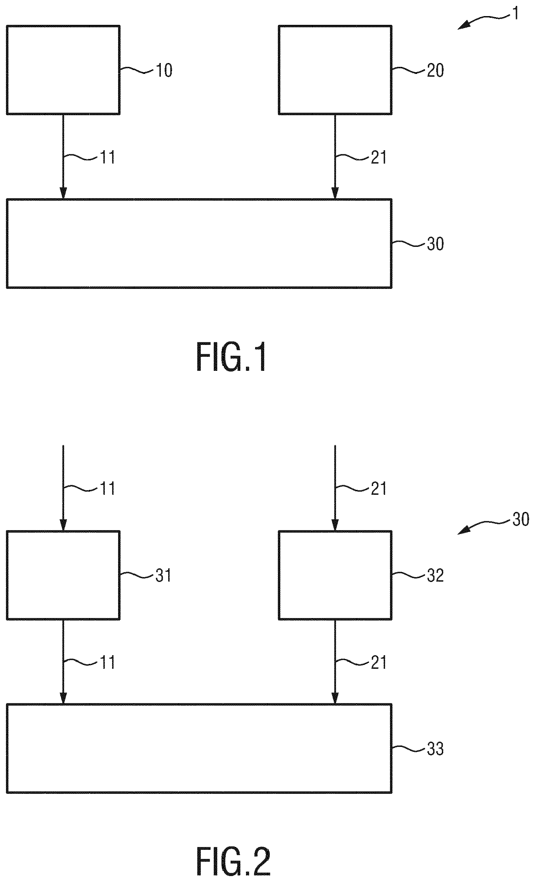

Fig. 1 shows a schematic diagram of a first embodiment of a system 1 for detecting atrial fibrillation of a subject according to the present invention. The system 1 comprises a radar sensor 10, a reference signal sensor 20 and device 30 for detecting atrial fibrillation of a subject.

The radar sensor 10 is configured to acquire a radar signal 11 when arranged at the subject's thorax, preferably close to the heart. The radar signal 11 represents radiation reflected from the subject's thorax in response to radiation emitted to the subject's thorax. The radar signals11 are preferably in a frequency range up to 10 GHz, e.g. 2.4 GHz, i.e. at a frequency that can enter sufficiently deep into the subject's body so that the radar signal 11 carries sufficient information on the heart movement and makes the heart movements "visible". Lower frequencies generally provide higher penetrations depths. The radar sensor 10 may e.g. be configured as a radar antenna, e.g. the antenna characteristic is optimized for use close to a body (for instance, according to the tissue characteristics such as conductivity, permittivity.

The reference signal sensor 20 is configured to acquire a reference signal 21 of the subject. The reference signal 21 represents either an electrocardiography (ECG) signal, or a photoplethysmography (PPG) signal, or an accelerometer (ACC) signal, whereby it is also possible that two or more reference signals of the same type or of different type (e.g. an ECG signal and an ACC signal) are used. Generally, the reference signal 21 is acquired synchronously with the radar signal 11.

The radar sensor 10 and the reference sensor(s) 20 may be arranged directly on and in contact with the skin (e.g. fixed by a strap or sticky tape) or may be held or arranged at a small distance (e.g. at a distance in the millimeter or centimeter range). The radar sensor 10 and the reference sensor(s) 20 may be provided and arranged as separate elements or may be integrated into one or more common sensor units.

The device 30 may generally be configured as processing element, e.g. as a processor or computer that is arranged at a separate location, e.g. in a doctor's office or as part of a patient monitor. It may also be represented by a processor of a user device, such as a smartphone, laptop or smart watch, which is programmed accordingly. In this case the radar signal 11 and the reference signal(s) 21 are transmitted to the device 30 in a wired or wireless manner, e.g. via cables from the respective sensors or via wireless transmitter included in the sensors, such as Bluetooth, NFC or ZigBee transmitters.

Alternatively, the device 30 may be combined with the sensors 10, 20 into a common wearable or handheld (mobile) device, which is mounted to the subject's thorax or which is held by the user or another person to the subject's thorax when being in use. In this case the common device may further comprise power supply means such as a (e.g. rechargeable) battery, an output unit such as a display and a user interface for enabling user control and user input.

Fig. 2 shows a schematic diagram of a first embodiment of a device 30 for detecting atrial fibrillation of a subject according to the present invention. The device 30 comprises a radar input 31 configured to obtain (i.e. receive or retrieve) the radar signal 11 acquired by the radar sensor 10, a signal input 32 configured to obtain (i.e. receive or retrieve) at least one reference signal 21 of the subject, and an analysis unit 33, e.g. a processing unit. To detect atrial fibrillation of the subject the analysis unit 33 identifies p-wave time intervals and/or ventricle contraction intervals in the reference signal and evaluates one or more p-wave sections of the radar signal corresponding to identified p-wave time intervals and/or one or more ventricle contraction sections of the radar signal corresponding to identified ventricle contraction intervals.

Conventional single-lead ECG based detection systems suffer from performance issues due to large false positive rates, which is due to difficult or impossible assessment of p-waves in single or two lead ECG based monitoring devices. These and other problems are overcome by the present invention. The present invention particularly enables an earlier detection of silent AF by the combination of at least two complementary sensing modalities and achieves a better sensitivity and specificity detecting AF compared to conventional techniques based on single-lead ECG measurements alone as well as a lower false positives rate. Further, the proposed solution enables improved performance to detect other heart arrhythmias with improved detection performance versus known methods and support patients during cardiac rehabilitation via objective measures of the patient's heart performance. Still further, the proposed system and device can be used to alarms and warn the patient as well as bystanders and emergencies in case of critical states. It can be implemented as an easy-to-use and easy-to-apply small wearable or handheld system for untrained person or as battery operated mobile device.

The claimed system can be implemented in various ways. Preferred embodiments are:
a) A wearable system comprising a single- or two-lead ECG sensor, an acceleration sensor and a Doppler radar sensor. The advantage of this embodiment is the use of the ECG signal as a first reference signal, which is commonly known to segment the various heart phases.
b) A wearable system comprising a PPG sensor (e.g. an optical contact sensor using a red LED, an infrared LED and a photosensor, as conventionally known in the art of PPG sensor technology), an acceleration sensor and a Doppler radar sensor. In contrast to embodiment a), no electrodes are needed, which is a key advantage for patient compliance due to a higher comfort. However, a reliable segmentation of the radar signal may require a more sophisticated strategy compared to the standardized ECG signal since the PPG signal does per se not contain the p-wave, but mainly the systolic and diastolic intervals can be clearly separated.
c) A wearable system comprising a single- or two-lead ECG sensor, a PPG sensor, an acceleration sensor and a Doppler radar sensor. This embodiment provides access to more hemodynamic surrogate measures, e.g. BP inference based on surrogate measures such as the pulse transit time / pulse arrival time including correction of the impact e.g. of the pre-ejection period.
d) A wearable system comprising an acceleration sensor and a Doppler radar sensor. Instead of a PPG sensor also an embodiment with only an acceleration sensor and the radar sensor is feasible, which is advantageous in terms of power consumption.

All components of the system can be integrated into a small wearable device, which can be fixed to the chest by ECG electrodes and if needed with additional adhesives. Further, a handheld device for spot check may implement the invention. As explained above, in an embodiment the system includes the sensors, processing means, power supply, data recording and connectivity means.

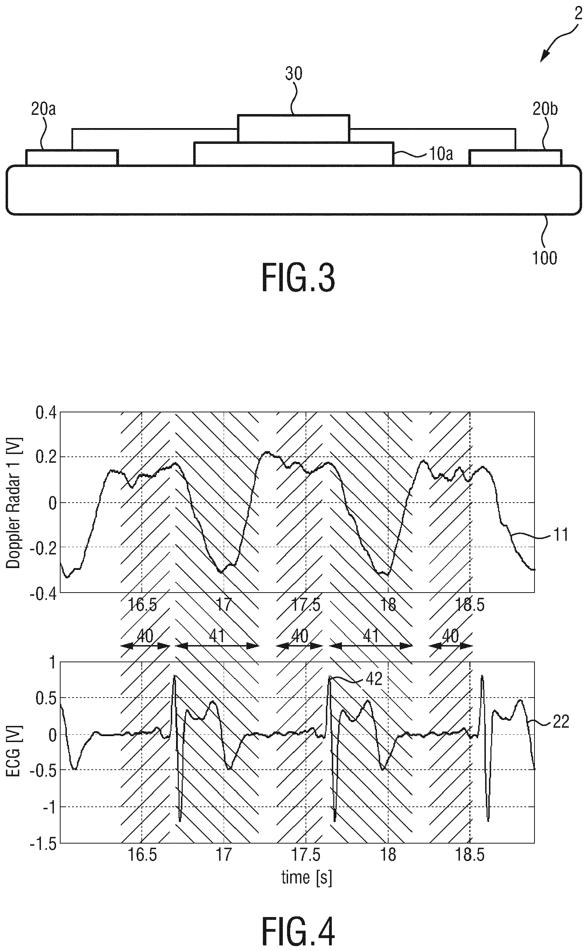

Fig. 3 shows a schematic diagram of a second embodiment of a system 2 for detecting atrial fibrillation of a subject according to the present invention. For optimized coupling of the radar sensor to the subject's body 100 a radar antenna 10a may be used that is integrated in a patch embodiment in order to ensure optimal coupling of the radio waves into the body. This is achieved by a material matching body properties versus requirements on antenna design. Further, two (or more) ECG electrodes 20a, 20b representing the reference sensor in this embodiment are attached to the body 100. The device 30 is integrated with the radar antenna 10a and the ECG electrodes 20a, 20b into a common wearable device.

In another embodiment, the radar antenna 10a and the ECG electrode(s) 20a, 20b may be combined in order to reduce the access points to the subject's body 100.

In the following various aspects of the present invention and embodiments of the claimed method are discussed in more detail.

According to the present invention Doppler radar signals obtained from the subject's chest that characterize heart wall movements are used to detect atrial fibrillation. Electromagnetic waves are reflected at boundary layers between areas of different electrical properties (permittivity and conductivity). This applies for the boundary between the heart wall and surrounding tissue. Relative motion between the transmitter and the receiver of a wave yields a frequency shift of the reflected wave as perceived by the receiver. This frequency shift is proportional to the velocity of the motion. The effect is called the Doppler effect.

A microwave transceiver emits a continuous-wave radar beam into the thorax and receives the reflection. The reflection of a wave at moving surfaces like the heart wall leads to a frequency shift in the reflected signal as described above. Wall motion abnormalities cause typical morphology changes of the Doppler radar sensor signal. There are several ways to analyze the signal changes. One possibility is to compare the radar signal to a previously recorded baseline signal and detect atrial fibrillation using the change in signal morphology. Another approach is to observe the changing of typical features of the radar signal such as the average signal energy of the first derivative or appearance as a measure of the heart wall velocity. An exemplary approach for observing changes of typical features of a radar signal can be found in J.A.J. Thijs, J. Muehlsteff, R. Pinter, The use of a two channel Doppler Radar Sensor for the characterization of heart motion phases, IEEE EMBC 2006. The method disclosed in this document may be applied here.

Fig. 4 shows a signal diagram of a radar signal 11 and a synchronously detected ECG signal 22, representing an exemplary reference signal, as used in the system shown in Fig. 3. These signals 11, 22 are obtained from the chest area of a healthy subject. Two regions 40, 41 are indicated, which mark the movement of the atrial wall (indicated as phases 40) and the ventricle movement (indicated as phases 41). Phases 40 represent the radar signatures during time periods of atrial contraction and are called p-wave sections (for the radar signal 11) and p-wave time intervals (for the reference signal, here the ECG signal 22). Phases 41 represent the radar signatures during time periods of ventricle contraction, with much larger velocity and signature, and are called ventricle contraction sections (for the radar signal 11) and ventricle contraction intervals (for the reference signal, here the ECG signal 22).

The two phases 40, 41 can be segmented via the analysis of the ECG signal 22 in respect to the R-peaks 42 and characteristic heart rate dependent periods in respect to the R-peak before and after the R-peak 42. For AF detection, the atrial movement is of particular interest.

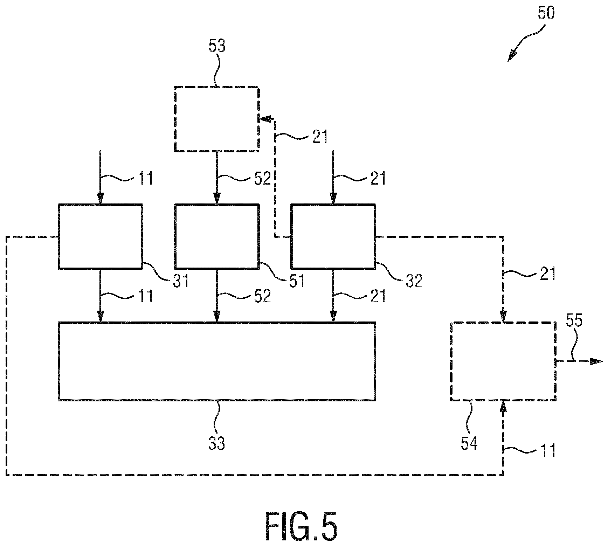

Radar signatures may depend on the posture of the subject, since the heart can move internally and therefore distances from the radar sensor to the heart changes and finally the radar signatures as well. As a consequence, to improve reliability of the detection, the posture may be included in the signal processing chain, as proposed in another embodiment of the device 50 schematically shown in Fig. 5, in which the a posture input 51 may be provided that is configured to obtain a posture signal 52 indicating posture of the subject during the acquisition of the radar signal 11, wherein said analysis unit 33 is configured to select, based on the obtained posture signal 52, a p-wave section template or baseline from a number of p-wave section templates or baselines for different postures. The posture signal 52, i.e. the information on the subject's current posture, may thus be generated by an external means, e.g. a posture sensor (not shown) as conventionally known. Alternatively, a posture determination unit 53 may additionally be provided that is configured to determine the posture signal 52 from an ACC signal obtained as reference signal by the signal input 32.

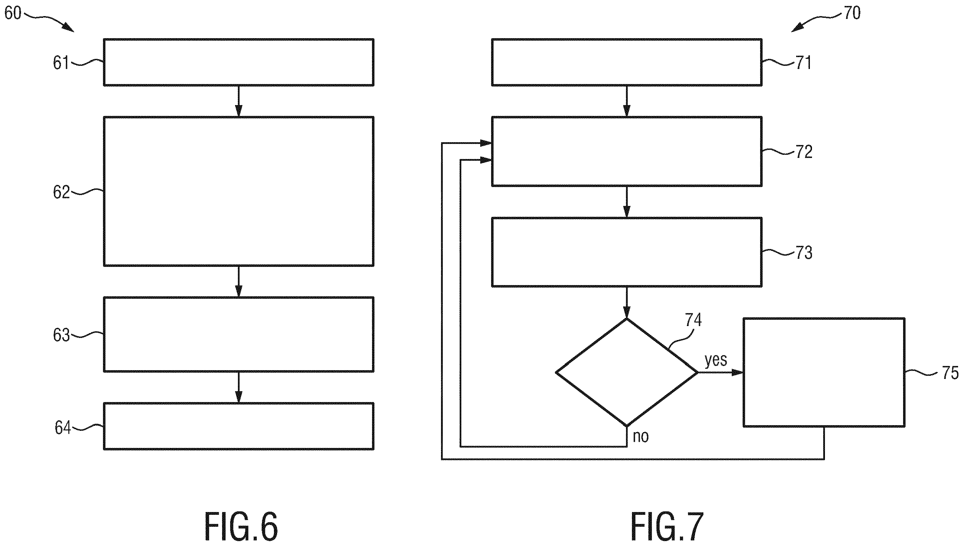

An example of an AF detection method 70 based on static templates, which have been acquired in a calibration method 60 shown in Fig. 6, e.g. conducted by the treating physician, is shown in Fig. 7.

In a first step 61 of the method 60 the calibration is started. In a second step 62 multi-lead ECG signals, a radar signal and an ACC signal are simultaneously acquired. A body location is chosen for placement of the sensors. Various body positions in the earth gravity field are taken and the acquired signals are logged versus the body posture and physical stress test induced heart rate changes. In step 63 radar signal templates are generated versus the body location, the ECG signatures and the body posture (obtained e.g. from the ACC signal). The generated radar signal templates are then stored so that they can be used during AF detection depending on the body posture and/or body location (where the sensors are placed).

In a first step 71 of the method 70 the monitoring period is started for AF detection. In a second step 72 single-lead (or multi-lead) ECG signals, a radar signal and an ACC signal are simultaneously acquired. In a third step 73 the segmented (stored) radar templates, as e.g. generated by the method 60, are compared to the real-time signals as acquired in step 72. In a fourth step 74 it is checked if a deviation, e.g. absolute differences or relative changes in particular segments of the signal or differences or relatives changes of parameters extracted from the signal morphology, is given in order to check for AF presence. If there is no deviation the method returns to step 72. If there is a deviation, in a fifth step 75 the detected AF event is logged and feedback (e.g. an alarm or warning or just information) is provided to the user and/or a caregiver. Further, input of the user may be requested, e.g. to indicate symptoms to verify if there is really an AF event or if there may be a detection or measurement failure. Then the method returns to step 72 as well to continue monitoring.

A higher specificity in order to discriminate AF from other common arrhythmias is achieved by analysis of the contraction phase 41 as well, which is also visible via the radar signal 11 as shown in Fig. 4. The same approaches as discussed above for AF detection apply to this signal processing as well.

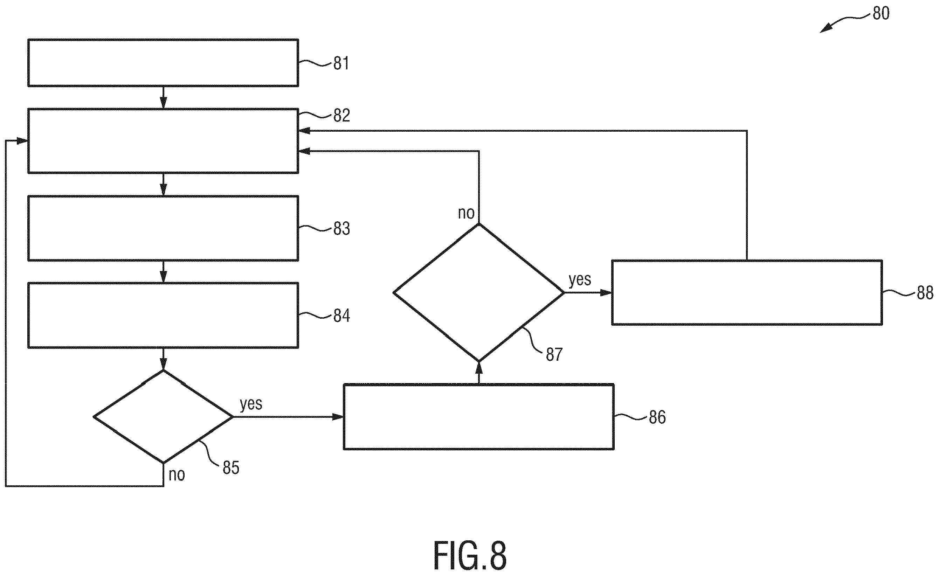

Another example of an AF detection method 80 based on dynamic templates is shown in Fig. 8. This method 80 can skip a dedicated calibration procedure by comparing radar signatures acquired continuously. This approach does not require a calibration step (i.e. a method as shown in Fig. 6) as it is needed for static templates of radar signatures.

In a first step 81 of the method 80 the monitoring period is started for AF detection. In a second step 82 single-lead (or multi-lead) ECG signals, a radar signal and an ACC signal are simultaneously acquired, from each of which a minimum time window is stored. In a third step 83 the acquired radar signal is segmented via periods in the ECG signal (heart rate dependent) and logged versus the body posture, if available. From the beat to beat intervals or other appropriate time periods of the ECG signal heart phases are detected in a fourth step 84. In a fifth step 85 it is checked if AF presence is probable. If not, the method returns to step 82. If yes, the radar signatures obtained during R-R intervals (or other time windows) are compared to previously acquired radar signatures in a sixth step 86. Then, it is checked in a seventh step 87 if significant changes of the radar signatures for constant body posture are detected. If no the method continues with step 82, otherwise in an eighth step 88 the AF event is logged and feedback (e.g. an alarm or warning or just information) is provided to the user and/or a caregiver. Further, input of the user may be requested, e.g. to indicate symptoms to accompanied with the detected AF event or if there may be a detection or measurement failure. Then the method returns to step 82 as well to continue monitoring.

Hence, according to this embodiment continuously acquired signals are segmented and compared with previous recordings e.g. from beat to beat or in other appropriate time periods. If e.g. an AF issue from inter-beat-interval analysis is suspected, the radar signatures are compared to deduce AF presence by comparing the current radar signal with previously acquired radar signals without expected AF.

Templates of radar signal during heart wall motion can also be obtained during calibration procedures carried out with standard hospital screening equipment like US imaging or other techniques used to analyze mechanical and hemodynamic activity of the heart. During such calibration process the radar signature is captured simultaneously with, for instance, US imaging during normal sinus rhythm. Radar signal features are then highlighted during atrial contraction and valve opening and closing and ventricular contraction so that fiducial points in the radar signal can be personalized and identified. Any deviation in time and amplitude of such fiducial points from the template in a follow up monitoring period will represent an index of compromised atrial contraction possibly due to AF.

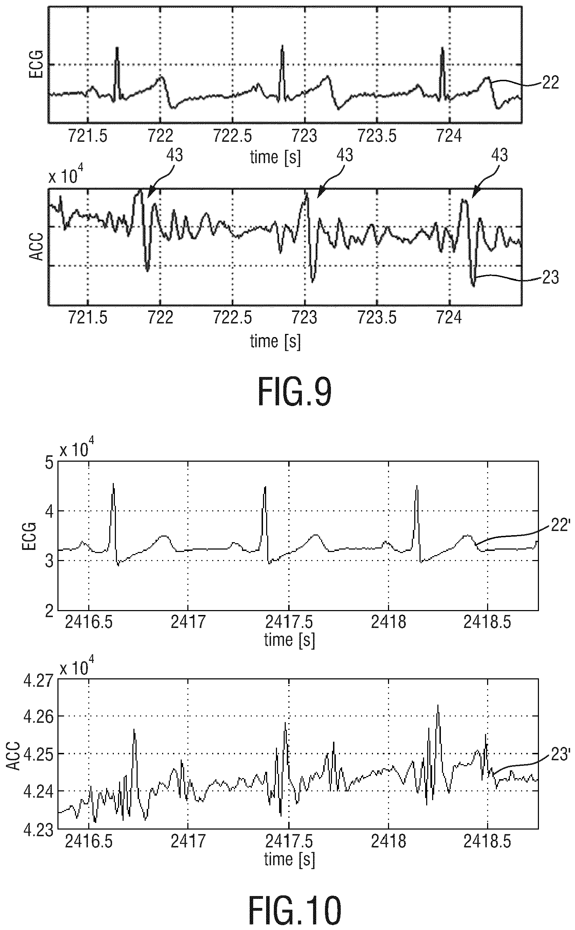

According to another embodiment the radar signal may be segmented by use of an ACC signal, in particular a chest acceleration signal. This is particularly useful if no ECG signal is available and/or if the use of ECG electrodes shall be avoided or is hardly possible. Fig. 9 shows an acquired chest acceleration signal 23 (ACC signal) representing a reference signal in this embodiment versus an ECG signal 22 (which is shown here only for comparison). In the ACC signal 23 the heart contractions 43 due to ventricle contraction can be easily seen so that ventricle contraction intervals can be identified. In contrast, atrial contraction typically do not have sufficient signal-to-noise ratio to be identified in ACC signals, so that p-wave time intervals are hard to segment in an ACC signal. Therefore, in this case the ventricle contraction from the ACC signal 23 is used for segmentation of the radar signal. Fig. 10 shows another example of an ECG signal 22' and an ACC signal 23' for illustrative purposes.

In another embodiment, both the ECG signal 22 and the ACC signal 23 may be used as reference signals to segment the p-wave intervals (from the ECG signal) and the ventricle contraction intervals from the ACC signal 23 and to use both of them to segment the corresponding sections in the radar signal, which are then evaluated to detect AF events.

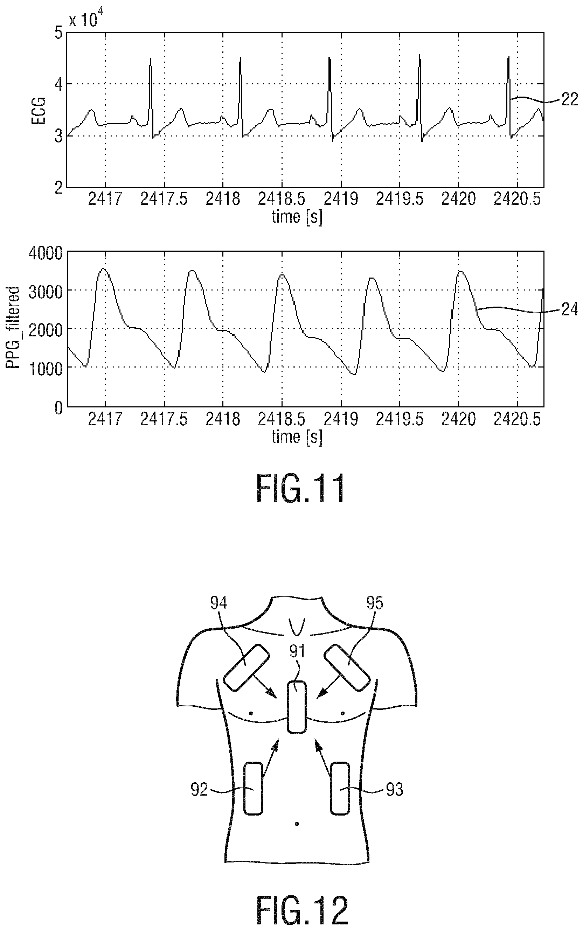

In another embodiment, PPG signals may be used as reference signals to segment the systolic and diastolic intervals, which are then used to segment the corresponding sections in the radar signal, which are then evaluated to detect AF events. Fig. 11 shows an exemplary PPG signal 24 representing a reference signal in this embodiment versus an ECG signal 22 (which is shown here only for comparison).

In still another embodiment, both the ECG signal 22 and the PPG signal 24 may be used as reference signals to segment the p-wave intervals (from the ECG signal) and the systolic and diastolic intervals as ventricle contraction intervals from the PPG signal 24 and to use both of them to segment the corresponding sections in the radar signal, which are then evaluated to detect AF events.

In a further embodiment of the present invention means are provided to support a user to find a recommended sensor location at the subject's thorax. This will be explained with reference to Fig. 12 that illustrates another embodiment of a device 90 according to the present invention enabling optimized placement of the sensors.

Radar characteristic signals depend on body location. This method supports a patient to find a pre-defined location at the upper chest, being identified e.g. by a treating physician, including a feedback option to find the recommended location easily himself.

First, in a calibration step, the recommended body location 91 is identified and stored e.g. by the treating cardiologist. The calibration procedure includes the acquisition of signals from other chest locations 91-95 being used as landmarks to provide signal difference features to determine the direction a sensor has to be moved towards the recommended location. This concept is enabled by the fact that the ECG signal and the radar signal are both location dependent including typical changes in direction and given body constraints. The ECG signal amplitude significantly differs by about a factor of 10 obtained from upper chest versus lower chest. Also, low or no respiration signals are observed from the radar sensor and different heart wall motion signals are obtained from various chest locations. These effects are used to guide to the recommended sensor location on the body.

An alternative is to use imaging technology, with which the preferred sensor location is documented via standard imaging processing and user feedback technologies by which the patient is guided when the sensor is being reapplied or a new sensor has to be attached.

In an embodiment a recommendation unit 54 (see Fig. 5) may optionally be provided that is configured to evaluate the obtained radar signals 11 and/or the obtained reference signals 21 to identify and recommend the optimum location of the subject's thorax for arrangement of the radar sensor and one or more reference, e.g. by issuing a recommendation notice 55 or showing the optimum location on a schematic drawing of a person's chest.

The present invention may be advantageously applied for cardiac rehabilitation, detection of AF with a reduced lead set at high detection performance, better self-management of AF patients, and new sensor modalities in clinical settings which may replace MRT, CT or US.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for detecting atrial fibrillation of a subject, said device comprising:
- a radar input (31) configured to obtain a radar signal acquired by a radar sensor arranged at the subject's thorax, said radar signal representing radiation reflected from the subject's thorax in response to radiation emitted to the subject's thorax,
- a signal input (32) configured to obtain at least one reference signal of the subject, said reference signal representing an electrocardiography, ECG, signal, a photoplethysmography, PPG, signal or an accelerometer, ACC, signal and being acquired synchronously with the radar signal,
- an analysis unit (33) configured to identify p-wave time intervals and/or ventricle contraction intervals in the reference signal and to evaluate one or more p-wave sections of the radar signal corresponding to identified p-wave time intervals and/or one or more ventricle contraction sections of the radar signal corresponding to identified ventricle contraction intervals to detect atrial fibrillation of the subject.

2. Device as claimed in claim 1,
wherein said reference signal is an ECG signal and said analysis unit (33) is configured to evaluate one or more p-wave sections of the radar signal by comparing the p-wave sections to a p-wave section template or baseline and by using changes in signal morphology and/or of typical features of the radar signal to detect atrial fibrillation of the subject.

3. Device as claimed in claim 2,
further comprising a posture input (51) configured to obtain a posture signal indicating posture of the subject during the acquisition of the radar signal,
wherein said analysis unit (33) is configured to select, based on the obtained posture signal, a p-wave section template or baseline from a number of p-wave section templates or baselines for different postures.

4. Device as claimed in claim 3,
further comprising a posture determination unit (53) configured to determine said posture signal from an ACC signal obtained as reference signal.

5. Device as claimed in claim 1,
wherein said reference signal is an ECG signal and said analysis unit (33) is configured to evaluate one or more p-wave sections of the radar signal by comparing a recent p-wave section to one or more earlier p-wave sections and by using changes in signal morphology and/or of typical features of the radar signal to detect atrial fibrillation of the subject.

6. Device as claimed in claim 1,
wherein said reference signal is a PPG signal or an ACC signal and said analysis unit (33) is configured to evaluate one or more ventricle contraction sections of the radar signal by comparing the ventricle contraction sections to a ventricle contraction section template or baseline and by using changes in signal morphology and/or of typical features of the radar signal to detect atrial fibrillation of the subject.

7. Device as claimed in claim 1,
wherein said reference signal is a PPG signal or an ACC signal and said analysis unit (33) is configured to evaluate one or more ventricle contraction sections of the radar signal by comparing a recent ventricle contraction section to one or more earlier ventricle contraction sections and by using changes in signal morphology and/or of typical features of the radar signal to detect atrial fibrillation of the subject.

8. Device as claimed in claim 6 or 7,
wherein said analysis unit (33) is configured to identify systolic phases and/or diastolic phases as ventricle contraction intervals in case the reference signal is a PPG signal and to identify contraction phases and/or valve opening phases and/or valve closure phases as ventricle contraction intervals in case the reference signal is an ACC signal.

9. Device as claimed in claim 1,
wherein said analysis unit is configured to identify p-wave time intervals in an ECG signal representing a first reference signal and to identify ventricle contraction intervals in a PPG signal or an ACC signal representing a second reference signal and to evaluate one or more p-wave sections of the radar signal corresponding to identified p-wave time intervals and one or more ventricle contraction sections of the radar signal corresponding to identified ventricle contraction intervals to detect atrial fibrillation of the subject.

10. Device as claimed in claim 1,
wherein said radar input (31) is configured to obtain radar signals acquired at different locations of the subject's thorax and/or said signal input (32) is configured to obtain reference signals acquired at different locations of the subject's thorax,
wherein said device further comprises a recommendation unit (54) configured to evaluate the obtained radar signals and/or the obtained reference signals to identify and recommend the optimum location of the subject's thorax for arrangement of the radar sensor for acquiring radar signals and one or more reference sensors for acquiring one or more reference signals for use in the detection of atrial fibrillation.

11. System for detecting atrial fibrillation of a subject, said system comprising:
- a radar sensor (10) configured to acquire a radar signal when arranged at the subject's thorax, said radar signal representing radiation reflected from the subject's thorax in response to radiation emitted to the subject's thorax,
- a reference signal sensor (20) configured to acquire a reference signal of the subject, said reference signal representing an electrocardiography, ECG, signal, a photoplethysmography, PPG, signal or an accelerometer, ACC, signal and being acquired synchronously with the radar signal,
- a device (30) as claimed in claim 1 for detecting atrial fibrillation of a subject based on the acquired radar signal and the acquired reference signal.

12. System as claimed in claim 11,
wherein said system is configured as wearable or handheld device that can be mounted to the subject's body or that can be held at the subject's body for detecting atrial fibrillation of the subject.

13. Method for detecting atrial fibrillation of a subject, said method comprising:
- obtaining a radar signal acquired by a radar sensor arranged at the subject's thorax, said radar signal representing radiation reflected from the subject's thorax in response to radiation emitted to the subject's thorax,
- obtaining a reference signal of the subject, said reference signal representing an electrocardiography, ECG, signal, a photoplethysmography, PPG, signal or an accelerometer, ACC, signal and being acquired synchronously with the radar signal,
- identifying p-wave time intervals and/or ventricle contraction intervals in the reference signal, and
- evaluating one or more p-wave sections of the radar signal corresponding to identified p-wave time intervals and/or one or more ventricle contraction sections of the radar signal corresponding to identified ventricle contraction intervals to detect atrial fibrillation of the subject.

14. Computer program comprising program code means for causing a computer to carry out the steps of the method as claimed in claim 13 when said computer program is carried out on the computer.
